# EUROPEAN PATENT APPLICATION

(11) **EP 4 300 396 A1**
(43) Date of publication of application: **03.01.2024**
(21) Application number: 23182148.9
(22) Date of filing: 28.06.2023
(51) Int. Cl.: G06Q 10/087, G06Q 50/10, G06Q 50/28

(54) **SYSTEMS, DEVICES, AND METHODS FOR MANAGEMENT OF MILK ALLOCATION**

(30) Priority: 29.06.2022 US 202263356730 P; 24.08.2022 LU 502702
(71) Applicant: Prolacta Bioscience, Inc., Duarte, CA 91010 (US)
(72) Inventor: FOURNELL, Joseph, Azusa (US)
(74) Representative: Latscha Schöllhorn Partner AG

(57) **Abstract**

A system for managing milk allocation includes a server and a database. The database stores, for each lot of milk product, lot information that includes a listing parameter of donors, and a lot contribution parameter of each donor. The system also includes a device that reads the lot information associated with a unit of milk product to be administered to the subject, the lot information being for a lot used to fill that unit. The server receives a predetermined feed volume and feed information for a feed administered to the subject, the feed being generated using the unit of milk product. The server calculates the feed contribution of each donor to the feed, and also calculates a cumulative feed contribution of each donor for that subject. When the cumulative feed contribution exceeds a threshold, the server transmits a notification to a user devices of the cumulative feed contribution.

## Description

### Background

Donated human milk is frequently administered to preterm and term infants in a NICU (neonatal intensive care unit). The milk may be sourced from as many as hundreds or even thousands of donors and then pooled, processed, and bottled for use in the NICUs. With nursing of an infant by a non-biological mother carrying significant societal weight, an infant being fed a pooled milk product as described here then is conceivably being associated with all the hundreds/thousands of donors who contributed to that milk product. Even greater weight can be attached to an infant-donor relationship if the infant ends up, over time, consuming a non-trivial portion of their feed from a single donor. However, given the potential scale of such an operation - large number of donors each donating a small amount of milk, the large volumes of pooled milk that is used to generate feed products, the small volume of feed typically administered to such infants, and the large number of such infants in NICUs, it is extremely challenging to track such donor-infant associations.

### Summary

Some aspects are directed to a system for managing milk allocation to a set of subjects including a server and a database connected to the server. The database is configured to store, for each lot of a set of lots of milk products, lot information that includes a listing parameter of a set of donors for that lot, and a lot contribution parameter of each donor of the set of donors to that lot. The system also includes a device connected to the server, the device including an optical scanner configured to read the lot information associated with a unit of milk product to be administered to the subject, the lot information being for a given lot from the set of lots used to fill that unit of milk. The server is configured to receive, from a user of the device, an indication of a predetermined feed volume, and to receive feed information for a feed administered to the subject, the feed being generated using the unit of milk product. The feed information includes an indication of type of feed administered to the subject, a volume of the feed administered to the subject, and the lot information read from the optical scanner. The server is further configured to calculate, based on the predetermined feed volume, the volume of the feed, the type of the feed, and the lot information, the feed contribution of each donor of the set of donors associated with that lot information to the feed administered to the subject. The server is further configured to calculate, based on the feed contribution and historical feed information for the subject, a cumulative feed contribution of each donor of the set of donors for that subject. The server is further configured to, when the cumulative feed contribution exceeds a predetermined threshold, transmit a notification to one or more user devices associated with users of the device, the notification including an indication of the cumulative feed contribution.

Some aspects are directed to a method for managing milk allocation to a set of subjects that includes reading, via an optical scanner of a device, lot information associated with a unit of milk product to be administered to the subject, the lot information being for a given lot from a set of lots used to fill that unit of milk. The lot information for each lot includes a listing parameter of a set of donors for that lot, and a lot contribution parameter of each donor of the set of donors to that lot. The method further includes receiving, from a user of the device, an indication of a predetermined feed volume, and receiving feed information for a feed administered to a subject, the feed being generated using the unit of milk product. The feed information includes an indication of type of feed administered to the subject, a volume of the feed administered to the subject, and the lot information read from the optical scanner. The method further includes calculating, via a processor of the device and/or a server coupled thereto, based on the predetermined feed volume, the volume of the feed, the type of the feed, and the lot information. The feed contribution of each donor of the set of donors is associated with that lot information to the feed administered to the subject. The method further includes when the cumulative feed contribution exceeds a predetermined threshold, transmitting a notification to one or more user devices associated with users of the device, the notification including an indication of the cumulative feed contribution.

Some aspects are directed to a non-transitory computer-readable storage medium storing instructions, the instructions when executed causing at least one processor to read, via an optical scanner of a device, lot information associated with a unit of milk product to be administered to the subject, the lot information being for a given lot from a set of lots used to fill that unit of milk. The lot information for each lot includes a listing parameter of a set of donors for that lot, and a lot contribution parameter of each donor of the set of donors to that lot. The instructions further include instructions to receive, from a user of the device, an indication of a predetermined feed volume, and receiving feed information for a feed administered to a subject, the feed being generated using the unit of milk product. The feed information includes an indication of type of feed administered to the subject, a volume of the feed administered to the subject, and the lot information read from the optical scanner. The instructions further include instructions to calculate, via a processor of the device and/or a server coupled thereto, based on the predetermined feed volume, the volume of the feed, the type of the feed, and the lot information. The feed contribution of each donor of the set of donors is associated with that lot information to the feed administered to the subject. The instructions further include instructions to, when the cumulative feed contribution exceeds a predetermined threshold, transmit a notification to one or more user devices associated with users of the device, the notification including an indication of the cumulative feed contribution.

### Brief description of the Drawings

The skilled artisan will understand that the drawings primarily are for illustrative purposes and are not intended to limit the scope of the inventive subject matter described herein. The drawings are not necessarily to scale; in some instances, various aspects of the inventive subject matter disclosed herein may be shown exaggerated or enlarged in the drawings to facilitate an understanding of different features. In the drawings, like reference characters generally refer to like features (e.g., functionally similar and/or structurally similar elements).
FIG. 1A illustrates an example system for management of milk allocation.
FIG. 1B illustrates an example setting for use of the compute device(s) of FIG. 1A.
FIG. 2 illustrates components of a compute device of FIG. 1A.
FIG. 3 illustrates how feed units are generated from lots populated by donor milk.
FIG. 4 illustrates an example interface for entry of feed information.

### Detailed Description of the Invention

All combinations of the foregoing concepts and additional concepts are discussed in greater detail below (provided such concepts are not mutually inconsistent) and are part of the inventive subject matter disclosed herein. In particular, all combinations of claimed subject matter appearing at the end of this disclosure are part of the inventive subject matter disclosed herein. The terminology used herein that also may appear in any disclosure incorporated by reference should be accorded a meaning most consistent with the particular concepts disclosed herein.

### Example System

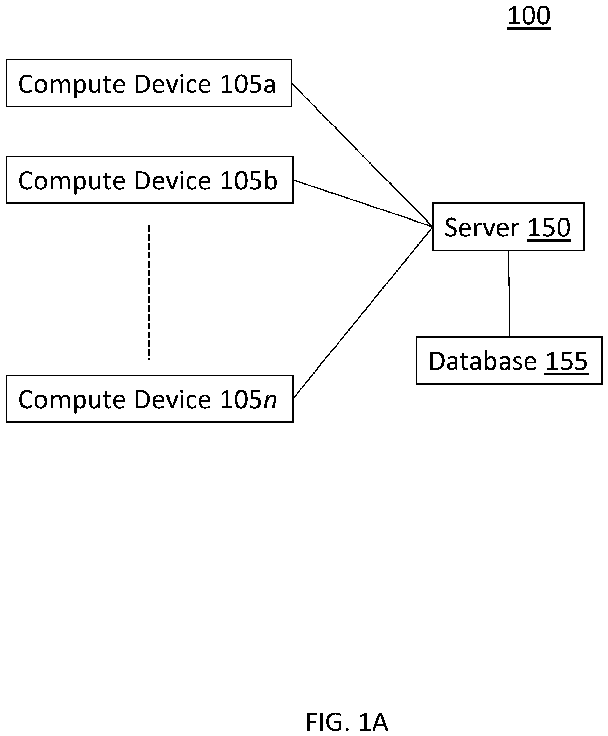

FIG. 1A illustrates an example system 100 useful for milk allocation, also sometimes referred to as a system for managing milk allocation parameters. Such parameters, as described in more detail herein, can include (but are not limited to) feed volume, lot contribution, donor contribution, lot number, and/or the like. The system 100 includes a set of compute devices 105a, 105b... 105*ₙ*, where n is any suitable number. The system also includes a server 150 communicably coupled to the devices 105a-105*n*. The system further includes a database 155 communicable coupled to the server 150. Each of the server 150 and the devices 105a-105n can include or encompass a processor and a memory/database that are communicably coupled with other. Each memory/database can encompass, for example, a random access memory (RAM), a memory buffer, a hard drive, a database, a programmable read-only memory (PROM), an erasable programmable read-only memory (EPROM), an electrically erasable read-only memory (EEPROM), a read-only memory (ROM), Flash memory, and/or so forth. The memory/database can store instructions to cause its corresponding processor to execute processes and/or functions as described herein. Each processor can be any suitable processing device configured to run and/or execute a set of instructions or code associated with the sensor/controller. The processor can be, for example, a general purpose processor, a Field Programmable Gate Array (FPGA), an Application Specific Integrated Circuit (ASIC), a Digital Signal Processor (DSP), and/or the like.

In some cases, the server 150 can be configured to provide on-demand computing services such as, for example, cloud computing services in a manner similar to Microsoft Azure, Amazon Web Services (AWS), ServerSpace, Google Cloud, IBM Cloud Services, Adobe Creative Cloud, Kamatera, VMware, Alibaba Cloud, Oracle, SAP, Salesforce, and/or the like. In some cases, each of the devices 105a-105*n* can be independently configured as a desktop, laptop, tablet, handheld/mobile device (including Smartphones), wearable device (including smart watches), and/or the like. All communications between the server 150 and the devices 105a-105*n* can be secure (e.g., encrypted) as is known in the art such as, for example, via HTTPS, symmetric or public/asymmetric key encryption, and/or the like.

In some cases, the server 150 and the devices 105a-105n can be configured as a multitenant architecture. Said another way, the server 150 can execute an instance of a software configured for managing milk allocation parameters as described herein, and each of the devices 105a-105n can be configured to share an instance of the software executing on the server. In other cases, the server 150 and the devices 105a-105n can be configured as a multi-instance architecture, with each device executing a separate instance of the software executing on the server. For example, the server 150 can execute the software as a SaaS (software as a service) offering, as a PaaS (platform as a service) offering, as a FaaS (function as a service) offering, and/or as a MBaaS (mobile backend as a service) offering.

During an example use, the devices 105a-105n can be useful for managing milk allocation parameters in a NICU (newborn intensive care unit) setting as explained herein, and be useful for managing milk allocation parameters for premature/preterm newborn infants being monitored and/or treated in the NICU. Generally, human milk is the food of choice for preterm and term infants because of its nutritional composition and immunologic benefits. The source of human milk can be, for example a donor or the infant's mother. The human milk can be fortified with various supplements, e.g., proteins, carbohydrates, minerals, and/or the like to meet the demanding and particular requirements for preterm infants. Fortifying human milk, whether it is the mother's milk or another donor's milk, is beneficial since milk from mothers who deliver significantly prematurely usually does not have adequate nutritional content to completely meet the increased metabolic and growth needs of their preterm infant relative to a full-term infant. Preterm infants can generally tolerate limited feeding volumes, so simply feeding the infant more human milk is not a viable solution. Accordingly, fortification of human milk with human milk-based fortifier allows for increased delivery of one or more human milk macronutrients (e.g., proteins, fats, carbohydrates) while minimizing the additional feeding volume, thereby providing additional, human milk-based nutrition in feedings that are tolerated by the premature infant.

When produced at an industrial scale, human milk and other such supplements (e.g., a fortifier for human milk with human or non-human (e.g., cow milk, soy milk) origin) are typically sourced from several donors (e.g., hundreds, or thousands) and combined, e.g., in large volume containers or vats. The fortification of the human milk with supplements can be performed before or after combination. The large volumes/vats of fortified milk can then be packaged into smaller units/bottles for single-use such as, for example, a 10 ml bottle, a 20 ml bottle, a 30 ml bottle, a 50 ml bottle, a 70 ml bottle, a 100 ml bottle, or a bottle containing more than 100 ml of fortified milk product, including all values and sub-ranges in between. Accordingly, each such unit can encompass milk contributed by each donor who contributed to the vat/lot it was poured from.

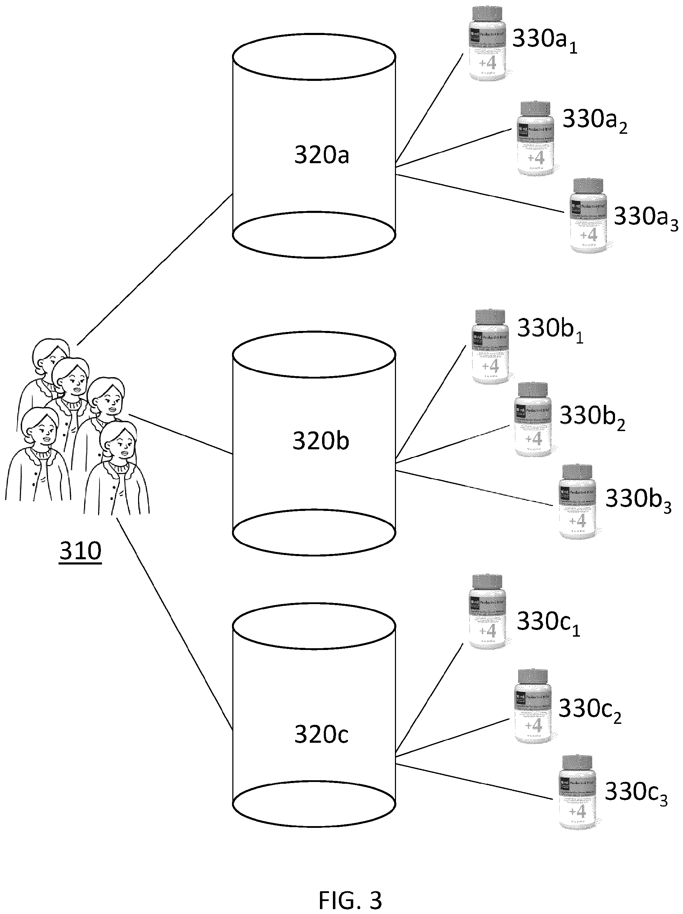

For example, FIG. 3 illustrates how, during production of the feed products, donors and/or providers 310 can contribute to one or more vats or lots 320a, 320b, and 320c. Each lot 320a-320c can hold, for example, 1000 liters, 1500 liters, 2000 liters, 2500 liters, 3000 liters, 3500 liters, 4000 liters or more, including all values and sub-ranges in between. Each lot 320a-320c can be bottled and/or packed into smaller units (e.g., the units 330a₁-330a₃ corresponding to the lot 320a) that are suitable for one-time use, such as for consumption or generation of a single feed, for example.

In some instances, it is desirable to track how much of the feed provided to a preterm infant from such units comes from a single donor. For example, in some cases, societal norms or laws may dictate that that an infant (preterm or otherwise) that nurses on some predetermined amount of milk from a single donor is in a milk kinship with that donor. Formation of such milk kinship can have societal and legal implications such as, for example, from fostering an allegiance between the infant's and that donor's families to deeming the infant and the donor as being immediate family. This can be desirable in some circumstances, and undesirable or even forbidden in other circumstances. For example, in some practices of Eastern Christianity, formation of such familial relations could prohibit marriage between kins of the infant's and that donor's families. As another example, per Islamic Law in some countries, milk kinship can be completely forbidden. In such cases, it is sometimes permissible for the infant to consume milk from a source other than its biological mother if it less than some predetermined amount such as, for example, less than some predetermined volume, less than some predetermined number of feeds, and/or the like.

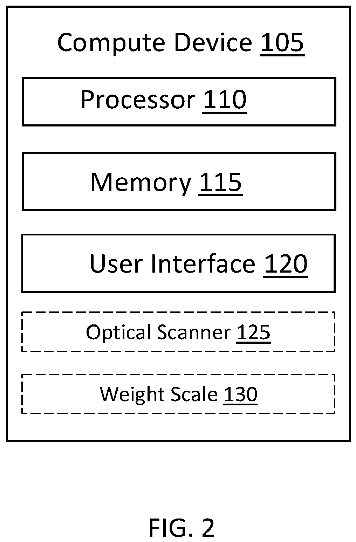

Aspects of the systems, devices, and methods as disclosed herein can hence be useful for monitoring milk allocation to a preterm infant and/or managing milk allocation parameters for that infant to avoid the formation of milk kinship between that infant and any particular donor. Such aspects are described with reference to operation of an example device 105 (see FIG. 2) that is representative of the devices 105a-105*n* for simplicity though it is understood that, as explained above, the functionality of the device 105 can also be imputed to the server 150 in part or in whole. Said another way, in some cases, the device 105 can be predominantly an interface to the functionality of the server 150.

As illustrated in FIG. 2, the device 105 can include a processor 110 and a memory 115 as described herein. The device 105 can further include a user interface 120 such as, for example, a display screen, a touch screen, a stylus, a keyboard, a mouse, combinations thereof, and/or the like, for users to interact with the device 105. The device 105 can optionally include (as illustrated in dotted lines in FIG. 2) or be couplable to (e.g., via a USB port of the device 105) an optical scanner/reader 125 that can be used to scan a machine readable code (e.g., a linear bar code, a matrix code such as a QR (quick response) code, and/or the like) on a milk unit that is to be administered to a specific subject, as described in greater detail later. The scanner 125 can include, but is not limited to, a pen-type reader, a laser scanner, a CCD reader, a camera (e.g., a smartphone camera), and/or the like. The device 105 can also optionally include or be couplable to a weight scale 130 that can be used for, for example, weighing a subject.

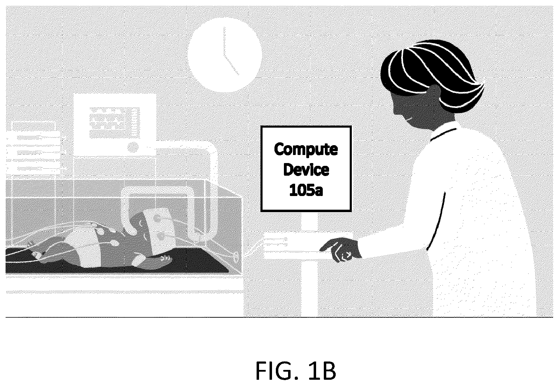

The device 105 can be generally disposed in a NICU as illustrated in FIG. 1B for use by, for example, a doctor and/or other healthcare professional of the NICU. The device 105 can be usable by a user (e.g., NICU staff) to enter feed information for one or more subjects, e.g., for one or more preterm infants in that NICU. The device 105 can be configured to register and maintain user accounts based on any suitable user information (e.g., name, phone number, email, role/designation, name of healthcare institution or hospital of the NICU, name/identifier of NICU unit of work, and/or the like) and to maintain authentication information (e.g., username and password) for each user. In some cases the device 105 can be configured to maintain an administrative account that can be used to manage the users such as, for example, add or remove users, change user privileges, etc. In some cases, users can only generate, view, and manage records for subjects at their NICU and/or institution (e.g., when the institution includes more than one NICUs). In some cases, the users can be blocked and/or otherwise prohibited from viewing records of subjects at other NICUs at their institution and/or at other institutions. In some cases, user privileges can be based on role based access control (RBAC) to permit authorized users to carry out one or more of these actions, attribute-based access control (ABAC) to permit users that have specific attributes (e.g., clearance) to carry out one or more of these actions, permission-based access control (PBAC) to permit specific user(s) to carry out specific tasks (e.g., edit weight of the subject, view the feed information but not other subject information), and/or combinations thereof.

### Subject Registration

The device 105 can be configured to permit a user to register a new subject (e.g., a preterm infant) for whom one or more milk allocation parameter(s) are to be managed. User registration information can include, but is not limited to, a name for the subject, the name(s) for one or more biological and/or legal parents of the subject, a date of birth for the subject, a birth weight of the subject, a biological sex of the subject, a birth head circumference of the subject, a gestational age of the subject, a start date for enteral feeds for the subject, a target daily weight gain for the subject (e.g., in kg/day), a target date or a target date range to transition the subject to cow milk-based products (e.g., at or after about 34 weeks post menstrual age), one or more of the users as associated with the subject, whether the subject is an active subject (for whom milk allocation is being currently managed) or has been discharged, and/or the like. In some cases, the weight of the subject can be provided as input via the weight scale 130.

In some cases, the registration information can include a specification of a subject identifier that is unique to the NICU and/or the institution that the users of the device 105 have visibility into. The specification of a subject identifier can be constrained in any suitable manner such as, for example, to have a minimum number of characters, to include or prohibit numerical characters, to include or prohibit one or more non-alphabet, non-numerical characters, and/or the like. In other cases, the device 105 can be configured to generate a unique subject identifier for each registered subject such as, for example, a universally unique identifier (UUID). In other cases, the subject identifier can be manually entered during registration of the subject such as, for example, to match a subject identifier for that subject being used throughout the institution. The registration information for all users and subjects can be stored locally at the device 105, at the server 150, in the database 155, and/or combinations thereof.

### Example Use

During use, the device 105 can be configured for daily and/or periodic operation as follows. For each subject registered with the device 105, users can be required to provide updated weight information for the subject daily. In some cases, the updated weight information can be provided via the weight scale 130, which in turn can be stored in the memory 115 as associated with that subject. In some cases, if the updated weight information is not provided by a certain time of day (e.g., by 8 pm), the device 105 can be configured (e.g., per computer executable instructions stored in the memory 115) to issue a notification such as, for example, a visual and/or audio notification through the device 105 itself, a text message or a prerecord phone call to a phone number of one or more users associated with the subject, and/or the like.

For each subject registered with the device 105, the device can be further configured (e.g., per computer executable instructions stored in the memory 115) to require that the user(s) provide updated feed information for the subject in a periodic manner such as, for example, for every feed, every day, every two days, every three days, more than three days, including all values and sub-ranges in between. In some cases, the device 105 can be further configured to require that the feed information be provided daily. In some cases, if the feed information is not provided by a certain time of day (e.g., by 8 pm), the device can be further configured to issue/transmit a notification as described above for updated feed information. In other cases, the device 105 can be configured to permit feed information to be provided more than once a day, which can account for the use of different milk products, different lots that the feed(s) were sourced from, and/or the like.

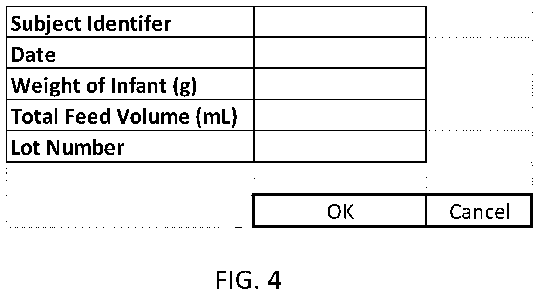

In some cases, the device 105 is configured to permit users to specify the volume of food/fortified milk that constitutes a single feed. In this manner, and for the purposes of determining milk kinship, feed volume can be tailored to local customs and/or laws such as, for example, indicating, via the interface of FIG. 4 (explained in greater detail later), a feed volume of 30 ml to comply with a local Fatwa that establishes feed limits to avoid development of kinship. In some cases, the device 105 can receive a specification of the feed volume from a central authority, such as the server 150. In some cases, the device 105 can encompass or include a Global Positioning System (GPS) module, such that it can determine its geographic location, and in turn use this location information to look up (e.g., locally on the device itself 105, by querying the server 150, and/or by querying the database 155) the feed volume (also sometimes referred to as a `predetermined feed volume' of, for example, 20 ml, 30 ml, 40 ml, 50 ml, and/or the like). In some cases, the device 105 can perform such a look up based on its IP address such as, for example, using a third-party IP tracker service. In some cases, one or more users (e.g., an administrative user/account) can override the device 105's determination of the feed volume via manual entry, to account for error in such determination.

In some cases, the feed information can include a specification of the type of feed the subject received. The type of feed can be selectable from predetermined options that include, but are not limited to, one or more types of human milk-based fortifiers (e.g., PROLACTPLUS^{™} Human Milk Fortifiers, e.g., HUMAVANT^{™}, PROLACT+4^{™}, PROLACT+6^{™}, PROLACT+8^{™}, PROLACT+10^{™}, SURGIFORT^{™}, Prolact CR^{®}), one or more types of complete human milk products (e.g., PROLACT20^{™}, NEO20^{™}, PROLACT HM^{®}, PREMIELACT^{®}, PROLACT RTF 24^{™}, PROLACT RTF 26^{™}, PROLACT RTF 28^{™}), milk from the biological mother of the subject, and/or the like. Non-limiting examples of type and volume of feed that can be input to the device 105 can include, but are not limited to, 30 ml PROLACT20^{™}, 20 ml of PROLACT HM^{®}, a feed that includes 10 ml of PROLACT+4^{™}, and/or the like.

In other cases, the lot information (described below) includes the specification of the type of feed, and the user will not be required or provided the option of entering feed type. More generally, a human milk-based fortifier can include any fortifier produced from and/or derived from human milk components. It is further understood that while various examples of feeds are listed herein, aspects of the systems, devices, and methods disclosed herein are useful for managing allocation of any substance that is administered to the subject(s).

In some cases, the feed information can include a specification of the total volume of feeding (e.g., in ml) the subject received that day. In some cases, the feed information can include a specification of the total volume of feeding (e.g., in ml) the subject received for a particular feed (see FIG. 4, explained in greater detail later).

In some cases, the feed information can include a specification of the lot that the feed was sourced from. This lot information can be provided in any suitable manner. For example, in some cases, the lot information can be included on a label of the bottle/unit used for that feed, and be entered manually by a user. The lot information can include, for example a lot identifier, which can be in any suitable format useful for uniquely identifying a lot of milk from which the unit was sourced. The device 105 can be configured to access locally stored information or the database 155 (e.g., directly or via the server 105) to extract the lot information based on the lot identifier. In other cases, the label of the unit can include a machine readable code (e.g., a barcode, a QR code, etc.) that can be read by the device 105 via the scanner 125, and in turn be used to extract the lot information. In some cases, since the lot information is read from each individual bottle/unit, the feed information can further include a bottle/unit identifier associated with that unit, which in turn can be employed for, for example, inventory management.

The lot information for a lot of a particular unit can include, but is not limited to, a specification of the type of feed, the total volume of the lot, the number of donors of the lot, a volume of contribution to the lot for each donor (also sometimes referred to as a lot contribution or a lot contribution parameter that is stored as part of the lot information in the database 155), a percentage/proportional contribution to the lot for each donor, an identification of each donor, combinations thereof, and/or the like. In some cases, the lot information includes at least donor identification information and the percentage contribution of each donor to that lot. For example, if a particular donor has contributed 10 liters to a lot of 1000 liters, the percentage contribution for that donor would be specified as 0.1%. In some cases, the donor identification information does not include any personally identifiable information (PII) such as names of the donors. In some cases, the donor identification information includes a unique donor identifier for each donor that is consistently used across different lots. An example input screen/interface is illustrated in FIG. 4.

In some cases, the feed information can also include updated subject information about the subject such as the subject's weight, length, head circumference, and/or the like, which can be used to update these fields in the registration information of the subject. In some cases, this updated subject information can be provided by the user of a different periodic basis such as, for example, weekly. In some cases, if the updated subject information is not entered within some predetermined time frame (e.g., within a week of the last update), a notification can be transmitted to one or more users associated with the subject as explained here.

Once the device 105 receives the feed information and the weight information for the subject, it can determine donor contribution to the subject's feed for that day in the following example manner, and based on the type of feed. The type of feed may include one or more fortifiers and/or a complete human milk product that can be administered without mixing. Generally, for example, if the type of feed includes a fortifier such as PROLACT+4^{™}, each donor's contribution by volume to that feed can be calculated based on a predetermined mixing ratio of PROLACT+4^{™} and human milk (which may be, for example, 1:4 or 20% per current specification), total feed volume, and percentage contribution of the donor. This can be generally represented as ((fortifier ratio * feed volume) * % donor contribution). As another example, if the type of feed is a fortifier such as PROLACT CR^{®}, each donor's contribution by volume to that feed can be calculated based on an assumed mixing ratio of PROLACT CR^{®} and human milk (e.g., about 125, or about 4%), total feed volume, and percentage contribution of the donor. This can be generally represented as ((0.04 * feed volume) * % donor contribution). In another example, if the type of feed includes the use of two or more different fortifiers, such as PROLACT+4^{™} and PROLACT CR^{®}, then it is possible that one or more donors may have provided milk for each of the two or more fortifiers. In such instances, the contribution of each donor who contributed milk to only one fortifier may be calculated as described above, and the contribution of donors who contributed milk to two or more fortifiers may be calculated as the sum of the contributions of each fortifier to the feed. As yet another example, if the type of feed is a complete human milk product that can be administered without mixing, each donor's contribution by volume to that feed can be calculated as the product of feed volume and percentage contribution by that donor.

For example, assume that the volume of a single feed is specified and/or otherwise predetermined to be 30 ml, and the subject received 150 ml of feed volume (i.e., five feeds) that includes PROLACT CR^{®} over the course of a day, with the donor contribution of each donor associated with the unit of PROLACT CR^{®} used (i.e., with the lot used to product that unit of PROLACT CR^{®}) being about 0.1%. The contribution of each donor to the feed for that subject for that day is then about 0.6 ml, or about 1/50^{th} of a single feed. Said another way, if the subject was continuously given feeds generated from units of PROLACT CR^{®} from the same lot (with the donor contribution of 0.1%), it would take about 50 feeds for the subject to receive one single feed of 30 ml from any given donor.

In some cases, the donor contribution can be based on the volume of donor milk contributed by the donor at the time of donation, irrespective of the feed type (e.g., a milk-based fortifier produced from the donor milk) consumed by the subject. In this manner, aspects disclosed herein can account for requirements (e.g., local customs/laws) that assess contribution of a donor to an initial pool of product, rather than contribution of the donor to a particular feed given to the subject. For example, consider that a donor contributed 10 liters to a lot of 1000 liters of donated milk. This lot is then used to product 500 liters of fortifier, i.e., the donor has effectively contributed 5 liters to the lot of fortifier. If a subject is then given a feed that includes 10 ml of the fortifier in 50 ml of feed (20% of feed), then even though the subject consumes 0.1 ml of the donor's contribution, that contribution came from 0.2 ml of the donor's original milk donation. Aspects disclosed herein can then deem the donor contribution to be that of the original donation (0.2 ml) or that actually consumed (0.1 ml) as configured. More generally, donor contribution can be based on the donor's contribution to a lot at any processing stage for that lot, and can account for how the donor's original contribution can be effectively increased or reduced due to processing.

Accordingly, the device 105 can be configured to track donor contributions to the feeding of a given subject over time by maintaining, for each subject, a table of donors (e.g., specified by their donor identifiers) as well as the cumulative volume of feed and/or number of feeds that subject has consumed from that donor till date. Per the example above then, after 50 days, the device 105 would determine that the subject has received 30 ml and/or one full feed from a particular donor (assuming that the subject was fed from the same lot for the 50 days), has received two full feeds after 100 days, and so on.

The device 105 can be further configured to display and/or otherwise output, for each subject, the listing of donors ordered from the highest cumulative feed volume and/or number of feeds to the lowest, such as to one of the users. The listing of donors can be stored as, for example, a listing parameter in the database 155. In some cases, the donor identifier is not displayed, and some other indicator (e.g., a serial number) is used to list the donors and their respective cumulative feed volume and/or number of feeds. In some cases, the device 105 can be further configured to display, to a user, on demand or when that user accesses a particular subject's profile, the subject identifier, and further display (for each day of feeding) the weight of the subject, total feed volume, the lot(s) used for the feeds for that day, and number of feeds by the donor with the highest number of feeds till that day. Table 1 illustrates an example display for a single feed type, and it is understood that the information of Table 1 can encompass the use and tracking of different feed types.

**Table 1**

| **Subject Identifier** | 123456 | | **Volume of a Feed (mL)** | | | 50 |
|---|---|---|---|---|---|---|
| | **Day 1** | **Day2** | **Day 3** | **Day 4** | **Day 5** | **Day 6** |
| **Weight of Infant (g)** | 1200 | 1218 | 1236 | 1255 | 1274 | 1293 |
| **Volume of Total Feed mL (Total volume feed to the patient, not the fortifer volume)** | 180 | 183 | 185 | 188 | 191 | 194 |
| **Lot Used** | RTF24-03 | RTF24-03 | RTF24-03 | RTF24-03 | RTF24 -03 | RTF24 -03 |
| **Number of Feeds by Highest Donor** | 1.05 | | | | | |

In some cases, the device 105 can be further configured to issue an alert if the number of feeds from any single donor (i.e., the donor with the highest number of feeds) exceeds one or more predetermined thresholds. For example, a first alert can be issued daily after the number of feeds from any single donor reaches 4 feeds. As another example, a second alert can be issued daily after the number of feeds from any single donor reaches 4.5 feeds. The second alert can be different from the first alert in color, intensity, volume, etc. to signify relatively greater urgency. As another example, a third alert can be issued daily after the number of feeds from any single donor reaches 5 feeds. Any suitable threshold(s) can be established based on local custom and/or law, and in some cases, the device 105 can be configured to permit one of the users to establish the threshold(s). Each alert can include, for example, a visual and/or audio notification through the device 105 itself, a text message or a prerecord phone call to a phone number of one or more users associated with the subject, and/or the like. In this manner, a user/caregiver can be empowered to take remedial action such as, for example, switching that subject to milk units from a different lot that does not include contributions from that donor.

Additional aspects and variations of the aspects disclosed herein are within the scope of the invention. For example, while explained herein in the context of milk allocation for preterm newborns, the systems, devices, and approaches disclosed herein can be useful for managing allocation of any substance that needs or is desired to be capped at a predetermined limit. In other cases, the systems, devices, and approaches disclosed herein can be useful for managing allocation of any substance that needs or is desired to be provided in some minimum amount, or within a desired range. For example, aspects disclosed herein can be used for ensuring a recipient receives some minimum number of calories when being administered nutrition. As another example, aspects disclosed herein can be used for ensuring a recipient receives some minimum amount of a substance such as protein or fat when being administered nutrition. As yet another example, aspects disclosed herein can be used for ensuring a recipient receives some minimum amount of a macronutrient such as protein, carbohydrates, or fat when being administered nutrition. As another example, aspects disclosed herein can be used for ensuring a recipient receives some minimum amount, but does not exceed some maximum amount, of a mineral such as (but not limited to) calcium, phosphorus, potassium, sodium, chloride, magnesium, iron, zinc, iodine, sulfur, cobalt, copper, fluoride, manganese, and/or selenium when being administered a supplement. As yet another example, aspects disclosed herein can be used for ensuring a recipient receives some minimum amount, but does not exceed some maximum amount, of a vitamin when being administered a multi-vitamin.

As another example, in some cases, aspects of the system shown herein such as the server 150, the device 105, and/or the database 155 can operate in concert to track the milk inventory for its associated NICU, such as by receiving inventory information in any suitable manner. For example, one or more users of the server 150 and/or device 105 can directly enter inventory information for each unit or for a set of units collectively that belong to the same lot into the device, e.g., via the interface 120. As another example, a set of milk units/bottles can be provided as a single package with a machine readable code that can be read by the device 105, such as via the scanner 125 of the device. As another example, the device 105 can receive the inventory information from or via the server 150, and can be further configured to (e.g., via operation of the processor 110) update the stored inventory information at or via the server as it receives daily feed information. As another example, the server 150 and/or device 105 can access the inventory information in the database 155, and can be further configured to (e.g., via operation of the processor 110) update the inventory information in the database as it receives daily feed information. In some cases, the server 150 and/or device 105 can be further configured to issue a notification as disclosed herein when the milk inventory falls below some predetermined threshold based on, for example, the number of subjects currently registered with the server 150 and/or device 105, remaining inventory for a particular lot, remaining inventory across multiple lots, and/or the like. For example, the notification can be processed by the processor 110 and delivered to the user via the interface 120. In some cases then, when the number of feeds for the donor with the highest number of feeds exceeds one or more predetermined thresholds for a given subject, the server 150 and/or device 105 can be configured to then recommend, based on the inventory information, that that subject be subsequently fed from a different lot that is available at its associated NICU.

In some cases, when the number of feeds for the donor with the highest number of feeds exceeds one or more predetermined thresholds for a given subject, the device 105 can be further configured to calculate the maximum number of remaining feeds that can be given to the subject from the same lot before exceeding the maximum permissible number of feeds from a single donor. In some cases, the maximum number of remaining feeds can be calculated based on the nature of the feed types being administered to the subject. Said another way, the maximum number of remaining feeds would likely be different if the subject was being administered a ready-to-feed product that includes human milk with one or more fortifiers, was being administered a fortifier, or some combination of both, and aspects disclosed herein can account for such a scenario.

As another example, the device 105 can be further configured to make preemptive recommendations for feeding a particular subject. For example, the device 105 can be configured such that upon entry of the subject identifier, data, feed type, and subject weight in the interface of FIG. 4, the device 105 can auto-populate the `total feed volume' field as a recommendation of the amount of feed that subject should receive that day. The recommendation can be based on, for example, the growth rate of the subject, which in some cases can be manually entered by the user. For example, the device 105 can monitor the subject weight entered over time and recommend a greater feed volume than before if the weight increase is less than 15 grams/kg of weight per day, or recommend a lower feed volume than before if the weight increase is more than 15 grams/kg of weight per day. As one example, the device 105 can be configured to calculate total feed volume as a product of the subject weight and a recommended feed volume specified in ml per kg of body weight per day. For example, for a subject weighing 2 kg with a recommended feed volume of 150 ml/kg/day, the device 105 can calculate the total feed volume as 2 * 150 = 300 mL for the entire day. In some cases, the device 105 can account for a total number of feeds per day (manually entered or predetermined) to calculate the feed volume per feeding. For example, if the subject is to be given eight feeds a day, the feed volume per feeding can be calculated as 300/8 = 37.5 ml per feeding.

The device 105 can be further configured to auto-populate the `lot number' field as a recommendation of which lot the unit(s) to be fed to that subject should be drawn from. The recommendation can be based on the inventory information and the historical feed information (i.e., and the associated cumulative donor contribution information) for that subject. In some cases, the user can override one or more of these recommendations to enter the actual feed volume and/or lot number administered to that subject.

Aspects of the system, devices, and methods disclosed herein can be further useful for performing analytics based on the feed information being input by the users, as well as on other information that can be input or requested regarding the subjects. Such analytics can be at the single device level (e.g., for subjects at a single NICU and/or NICUs of a particular hospital or medical entity) and/or encompass multiple devices 105a-105n (e.g., for subject within a particular geographical location). Such analytics can include, but are not limited to determining, calculating and/or monitoring birth rates, growth rates, head circumference (including but not limited to head circumference at birth), body length (including but not limited to birth length)birth weights, weight(s) at discharge, gestational age, nutrition intake, mass balance as can be determined by weighing diapers of preterm infants, feeding type (e.g., bolus vs continuous), feeding information for when total parental nutrition (TPN) is employed, and/or the like, including performing statistical analysis on one or more of these factors. One example of such analytics is to determine a growth rate/velocity for the subject by comparing the subject's actual growth rate (i.e., based on the periodically updated subject information) to the target growth rate received at the time of registration. In some cases, the analytics, or a portion thereof, are performed at the server 150.

Aspects of the system, devices, and methods disclosed herein can be further useful for accounting for sibling relationships between the subjects. For example, the device 105 and/or the server 150 can be configured to permit, after registration, linking of two or more of the subjects as sibling such as, for example, identical or fraternal twins or other multiples. As an example, the device 105 can be configured to recommend that both twins be fed from the same lot to prevent inadvertent differences in development of familial ties with donors. As another example, the device 105 and/or the server 150 can be further configured to monitor weight gain for sibling as a way of studying what factors may affect weight gain other than genetic differences, since genetic differences can be ruled out for identical twins.

While generally explained herein with respect to preterm infants, it is understood that allocation of milk and/or other resources to other patient populations is within the aspects of the systems, devices, and methods disclosed herein. One example is infants with increased energy demands and/or needing limited fluid intake. For example, full-term infants born with congenital defects such as (but not limited to) congenital heart defects, congenital intestinal defects such as omphalocele or gastroschisis, and/or the like can benefit from the disclosed approaches.

Aspects disclosed herein can encompass a non-transitory computer-readable storage medium storing instructions that when executed can cause at least one processor (e.g., the processor 110) to carry out steps for determining milk allocation and/or managing milk allocation parameters as disclosed herein. Aspects disclosed herein can encompass a computer-readable storage medium comprising instructions which, when executed by one or more computers (e.g., the server 150 and/or the device 105) can cause the computer(s) to carry out steps for determining milk allocation and/or managing milk allocation parameters as disclosed herein.

While various inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize or be able to ascertain, using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

Also, various inventive concepts may be embodied as one or more methods, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

Embodiments of the present disclosure include the following examples.

Embodiment 1 includes a system for managing milk allocation to a set of subjects, comprising: a server; a database connected to the server, the database configured to store, for each lot of a set of lots of milk products, lot information including: a listing parameter of a set of donors for that lot; and a lot contribution parameter of each donor of the set of donors to that lot; and a device connected to the server, the device including an optical scanner configured to read the lot information associated with a unit of milk product to be administered to the subject, the lot information being for a given lot from the set of lots used to fill that unit of milk, wherein the server is configured to: receive, from a user of the device, an indication of a predetermined feed volume; receive feed information for a feed administered to the subject, the feed being generated using the unit of milk product, the feed information including: an indication of type of feed administered to the subject; a volume of the feed administered to the subject; and the lot information read from the optical scanner; calculate, based on the predetermined feed volume, the volume of the feed, the type of the feed, and the lot information, the feed contribution of each donor of the set of donors associated with that lot information to the feed administered to the subject; calculate, based on the feed contribution and historical feed information for the subject, a cumulative feed contribution of each donor of the set of donors for that subject; and when the cumulative feed contribution exceeds a predetermined threshold, transmit a notification to one or more user devices associated with users of the device, the notification including an indication of the cumulative feed contribution.

Embodiment 2 includes the system of embodiment 1, wherein the predetermined threshold is from about three times the predetermined feed volume to about five times the predetermined feed volume.

Embodiment 3 includes the system of embodiments 1 or 2, wherein the server is further configured to: receive, from the one or more users of the device, registration information for the subject; and calculate the cumulative feed contribution based on, for each day starting from or after a day of receiving the registration information, a feed contribution of each donor of the set of donors for one or more feeds administered to the subject on that day.

Embodiment 4 includes the system of any of embodiments 1 to 3, wherein the server is further configured to transmit the notification daily while the cumulative feed contribution on that day is equal to or above the predetermined threshold.

Embodiment 5 includes the system of any of embodiments 1 to 4, wherein the type of feed is selectable from a predetermined set of types of feed including a complete milk product that includes one or more fortifiers.

Embodiment 6 includes the system of any of embodiments 1 to 5, wherein the type of feed is a fortifier, and wherein the server and device are further collectively configured to calculate the feed contribution of each donor of the set of donors based on a predetermined mixing ratio for that fortifier.

Embodiment 7 includes the system of any of embodiments 1 to 6, wherein the device is a first device of a set of devices of the system, and wherein the server and the set of devices are configured as a multitenant architecture.

Embodiment 8 includes a method for managing milk allocation to a set of subjects, comprising: reading, via an optical scanner of a device, lot information associated with a unit of milk product to be administered to the subject, the lot information being for a given lot from a set of lots used to fill that unit of milk, the lot information for each lot of a set of lots of milk products including: a listing parameter of a set of donors for that lot; and a lot contribution parameter of each donor of the set of donors to that lot; receiving, from a user of the device, an indication of a predetermined feed volume; receiving feed information for a feed administered to a subject, the feed being generated using the unit of milk product, the feed information including: an indication of type of feed administered to the subject; a volume of the feed administered to the subject; and
the lot information read from the optical scanner; calculating, via a processor of the device and/or a server coupled thereto, based on the predetermined feed volume, the volume of the feed, the type of the feed, and the lot information, the feed contribution of each donor of the set of donors associated with that lot information to the feed administered to the subject; calculating, via the processor, based on the feed contribution and historical feed information for the subject, a cumulative feed contribution of each donor of the set of donors for that subject; and when the cumulative feed contribution exceeds a predetermined threshold, transmitting a notification to one or more user devices associated with users of the device, the notification including an indication of the cumulative feed contribution.

Embodiment 9 includes the method of embodiment 8, wherein the predetermined threshold is from about three times the predetermined feed volume to about five times the predetermined feed volume.

Embodiment 10 includes he method of embodiments 8 or 9, further comprising: receiving, from the one or more users of the device, registration information for the subject; and calculating the cumulative feed contribution based on, for each day starting from or after a day of receiving the registration information, a feed contribution of each donor of the set of donors for one or more feeds administered to the subject on that day.

Embodiment 11 includes the method of any of embodiments 8-10, further comprising transmitting the notification daily while the cumulative feed contribution on that day is equal to or above the predetermined threshold.

Embodiment 12 includes the method of any of embodiments 8-11, wherein the type of feed is selectable from a predetermined set of types of feed including a complete milk product that includes one or more fortifiers.

Embodiment 13 includes the method of any of embodiments 8-12, wherein the type of feed is a fortifier, the calculating the feed contribution of each donor of the set of donors being further based on a predetermined mixing ratio for that fortifier.

Embodiment 14 includes a non-transitory computer-readable storage medium storing instructions, the instructions when executed causing at least one processor to: read, via an optical scanner of a device, lot information associated with a unit of milk product to be administered to the subject, the lot information being for a given lot from a set of lots used to fill that unit of milk, the lot information for each lot of a set of lots of milk products including: a listing parameter of a set of donors for that lot; and a lot contribution parameter of each donor of the set of donors to that lot; receive, from a user of the device, an indication of a predetermined feed volume; receive feed information for a feed administered to a subject, the feed being generated using the unit of milk product, the feed information including: an indication of type of feed administered to the subject;
a volume of the feed administered to the subject; and the lot information read from the optical scanner; calculate, based on the predetermined feed volume, the volume of the feed, the type of the feed, and the lot information, the feed contribution of each donor of the set of donors associated with that lot information to the feed administered to the subject; calculate, based on the feed contribution and historical feed information for the subject, a cumulative feed contribution of each donor of the set of donors for that subject; and when the cumulative feed contribution exceeds a predetermined threshold, transmit a notification to one or more user devices associated with users of the device, the notification including an indication of the cumulative feed contribution.

Embodiment 15 includes the non-transitory computer-readable storage medium of embodiment 14, wherein the predetermined threshold is from about three times the predetermined feed volume to about five times the predetermined feed volume.

Embodiment 16 includes the non-transitory computer-readable storage medium of embodiments 14 or 15, further storing instructions that when executed cause the at least one processor to: receive, from the one or more users of the device, registration information for the subject; and calculate the cumulative feed contribution based on, for each day starting from or after a day of receiving the registration information, a feed contribution of each donor of the set of donors for one or more feeds administered to the subject on that day.

Embodiment 17 includes the non-transitory computer-readable storage medium of any of embodiments 14-16, further storing instructions that when executed cause the at least one processor to transmit the notification daily while the cumulative feed contribution on that day is equal to or above the predetermined threshold.

Embodiment 18 includes the non-transitory computer-readable storage medium of any of embodiments 14-17, wherein the type of feed is selectable from a predetermined set of types of feed including a complete milk product that includes one or more fortifiers.

Embodiment 19 includes the non-transitory computer-readable storage medium of any of embodiments 14-18, wherein the type of feed is a fortifier, further storing instructions that when executed cause the at least one processor to calculate the feed contribution of each donor of the set of donors further based on a predetermined mixing ratio for that fortifier.

## Claims

1. A system for managing milk allocation to a set of subjects, comprising:
a server;
a database connected to the server, the database configured to store, for each lot of a set of lots of milk products, lot information including:
a listing parameter of a set of donors for that lot; and
a lot contribution parameter of each donor of the set of donors to that lot; and
a device connected to the server, the device including an optical scanner configured to read the lot information associated with a unit of milk product to be administered to the subject, the lot information being for a given lot from the set of lots used to fill that unit of milk, wherein the server is configured to:
receive, from a user of the device, an indication of a predetermined feed volume;
receive feed information for a feed administered to the subject, the feed being generated using the unit of milk product, the feed information including:
an indication of type of feed administered to the subject;
a volume of the feed administered to the subject; and
the lot information read from the optical scanner;
calculate, based on the predetermined feed volume, the volume of the feed, the type of the feed, and the lot information, the feed contribution of each donor of the set of donors associated with that lot information to the feed administered to the subject;
calculate, based on the feed contribution and historical feed information for the subject, a cumulative feed contribution of each donor of the set of donors for that subject; and
when the cumulative feed contribution exceeds a predetermined threshold, transmit a notification to one or more user devices associated with users of the device, the notification including an indication of the cumulative feed contribution.

2. The system of claim 1, wherein the predetermined threshold is from about three times the predetermined feed volume to about five times the predetermined feed volume.

3. The system of claims 1 or 2, wherein the server is further configured to:
receive, from the one or more users of the device, registration information for the subject; and
calculate the cumulative feed contribution based on, for each day starting from or after a day of receiving the registration information, a feed contribution of each donor of the set of donors for one or more feeds administered to the subject on that day.

4. The system of any of claims 1 to 3, wherein the server is further configured to transmit the notification daily while the cumulative feed contribution on that day is equal to or above the predetermined threshold.

5. The system of any of claims 1 to 4, wherein the type of feed is selectable from a predetermined set of types of feed including a complete milk product that includes one or more fortifiers.

6. The system of any of claims 1 to 5, wherein the type of feed is a fortifier, and wherein the server and device are further collectively configured to calculate the feed contribution of each donor of the set of donors based on a predetermined mixing ratio for that fortifier.

7. The system of any of claims 1 to 6, wherein the device is a first device of a set of devices of the system, and wherein the server and the set of devices are configured as a multitenant architecture.

8. A method for managing milk allocation to a set of subjects, comprising:
reading, via an optical scanner of a device, lot information associated with a unit of milk product to be administered to the subject, the lot information being for a given lot from a set of lots used to fill that unit of milk, the lot information for each lot of a set of lots of milk products including:
a listing parameter of a set of donors for that lot; and
a lot contribution parameter of each donor of the set of donors to that lot;
receiving, from a user of the device, an indication of a predetermined feed volume;
receiving feed information for a feed administered to a subject, the feed being generated using the unit of milk product, the feed information including:
an indication of type of feed administered to the subject;
a volume of the feed administered to the subject; and
the lot information read from the optical scanner;
calculating, via a processor of the device and/or a server coupled thereto, based on the predetermined feed volume, the volume of the feed, the type of the feed, and the lot information, the feed contribution of each donor of the set of donors associated with that lot information to the feed administered to the subject;
calculating, via the processor, based on the feed contribution and historical feed information for the subject, a cumulative feed contribution of each donor of the set of donors for that subject; and
when the cumulative feed contribution exceeds a predetermined threshold, transmitting a notification to one or more user devices associated with users of the device, the notification including an indication of the cumulative feed contribution.

9. The method of claim 8, wherein the predetermined threshold is from about three times the predetermined feed volume to about five times the predetermined feed volume.

10. The method of claims 8 or 9, further comprising:
receiving, from the one or more users of the device, registration information for the subject; and
calculating the cumulative feed contribution based on, for each day starting from or after a day of receiving the registration information, a feed contribution of each donor of the set of donors for one or more feeds administered to the subject on that day.

11. The method of any of claims 8-10, further comprising transmitting the notification daily while the cumulative feed contribution on that day is equal to or above the predetermined threshold.

12. The method of any of claims 8-11, wherein the type of feed is selectable from a predetermined set of types of feed including a complete milk product that includes one or more fortifiers.

13. The method of any of claims 8-12, wherein the type of feed is a fortifier, the calculating the feed contribution of each donor of the set of donors being further based on a predetermined mixing ratio for that fortifier.

14. A non-transitory computer-readable storage medium storing instructions, the instructions when executed causing at least one processor to:
read, via an optical scanner of a device, lot information associated with a unit of milk product to be administered to the subject, the lot information being for a given lot from a set of lots used to fill that unit of milk, the lot information for each lot of a set of lots of milk products including:
a listing parameter of a set of donors for that lot; and
a lot contribution parameter of each donor of the set of donors to that lot;
receive, from a user of the device, an indication of a predetermined feed volume;
receive feed information for a feed administered to a subject, the feed being generated using the unit of milk product, the feed information including:
an indication of type of feed administered to the subject;
a volume of the feed administered to the subject; and
the lot information read from the optical scanner;
calculate, based on the predetermined feed volume, the volume of the feed, the type of the feed, and the lot information, the feed contribution of each donor of the set of donors associated with that lot information to the feed administered to the subject;
calculate, based on the feed contribution and historical feed information for the subject, a cumulative feed contribution of each donor of the set of donors for that subject; and
when the cumulative feed contribution exceeds a predetermined threshold, transmit a notification to one or more user devices associated with users of the device, the notification including an indication of the cumulative feed contribution.

15. The non-transitory computer-readable storage medium of claim 14, wherein the predetermined threshold is from about three times the predetermined feed volume to about five times the predetermined feed volume.

16. The non-transitory computer-readable storage medium of claims 14 or 15, further storing instructions that when executed cause the at least one processor to:
receive, from the one or more users of the device, registration information for the subject; and
calculate the cumulative feed contribution based on, for each day starting from or after a day of receiving the registration information, a feed contribution of each donor of the set of donors for one or more feeds administered to the subject on that day.

17. The non-transitory computer-readable storage medium of any of claims 14-16, further storing instructions that when executed cause the at least one processor to transmit the notification daily while the cumulative feed contribution on that day is equal to or above the predetermined threshold.

18. The non-transitory computer-readable storage medium of any of claims 14-17, wherein the type of feed is selectable from a predetermined set of types of feed including a complete milk product that includes one or more fortifiers.

19. The non-transitory computer-readable storage medium of any of claims 14-18, wherein the type of feed is a fortifier, further storing instructions that when executed cause the at least one processor to calculate the feed contribution of each donor of the set of donors further based on a predetermined mixing ratio for that fortifier.
